Europäisches Patentamt

(19) European Patent Office

Office européen des brevets

(11) Numéro de publication: **0 214 070**
**B1**

## (12) FASCICULE DE BREVET EUROPEEN

(45) Date de publication du fascicule du brevet: **16.01.91**

(51) Int. Cl.⁵: **A 61 L 27/00**

(21) Numéro de dépôt: **86420207.2**

(22) Date de dépôt: **29.07.86**

(54) **Produit de remplacement de la matrice osseuse favorisant l'ostéogénèse.**

(30) Priorité: **30.07.85 FR 8512053**

(43) Date de publication de la demande:
**11.03.87 Bulletin 87/11**

(45) Mention de la délivrance du brevet:
**16.01.91 Bulletin 91/03**

(84) Etats contractants désignés:
**AT BE CH DE FR GB IT LI LU NL SE**

(56) Documents cités:
**EP-A-0 030 583**
**FR-A-2 318 189**
**FR-A-2 350 826**

(73) Titulaire: **BIOETICA, Société Anonyme**
**51 rue Clément Marot**
**F-69007 Lyon (FR)**

(72) Inventeur: **Huc, Alain**
**96 chemin des Fonds**
**F-69110 Sainte Foy Les Lyon (FR)**
Inventeur: **Bejui, Jacques**
**52 rue Lieutenant Colonel Prévost**
**F-69006 Lyon (FR)**
Inventeur: **Allard, Roland**
**53 rue du Général de Gaulle**
**F-42400 Saint Chamond (FR)**

(74) Mandataire: **Maureau, Pierre**
**Cabinet GERMAIN & MAUREAU Le Britannia -**
**Tour C 20, Boulevard E. Déruelle**
**F-69003 Lyon (FR)**

Courier Press, Leamington Spa, England.

## Description

La présente invention concerne un matériau de substitution de la matrice osseuse, favorisant l'ostéogénese.

La perte de substance osseuse primitive ou secondaire a toujours posé des problèmes de reconstruction au chirurgien orthopédiste. Le capital osseux de l'homme n'est pas inépuisable même si des prélèvements peuvent être réalisés sur des zones donneuses sans préjudice mécanique: crêtes iliaques, épiphyses et métaphyses fémorales et tibiales, par exemple.

Les os de banques d'origine humaine ou animale posent des problèmes de réalisation technique et leur devenir fonctionnel n'est pas toujours mécaniquement satisfaisant. C'est pourquoi, on s'est efforcé de rechercher des matériaux de substitution d'origine plus ou moins organique. Ceux-ci peuvent être soit définitifs, soit temporaires. Dans le premier cas, ils autorisent une colonisation cellulaire, alors qu'ils apportent une résistance mécanique initiale non négligeable (coraux par exemple). Dans le deuxième cas, ils font progressivement place à un tissu ostéoïde susceptible de prendre les caractères mécaniques et histologiques du tissu osseux avec le temps et les contraintes.

On sait que, l'os, forme rigide de tissu conjonctif, est formé de cellules et d'une matrice extracellulaire. Cette matrice est essentiellement constituée de fibres de collagène, de protéoglycanes et d'une partie minérale. La partie protéique renferme principalement du collagène de type 1, très réticulé. Cette réticulation importante rend impossible toute extraction de ce collagène sans une attaque enzymatique préalable. La partie mucopolysaccharidique est composée essentiellement d'acide chondroïtine-4-sulfate et, dans une moindre mesure, d'acide chondroïtine-6-sulfate et d'acide hyaluronique. La partie minérale renferme environ 85% d'hydroxyapatite, du carbonate de calcium et de faibles quantités de fluorures de calcium et de magnésium. Cette matrice a deux fonctions: d'abord un rôle mécanique de soutien et un rôle biologique en constituant un milieu de choix pour la vie et le développement des cellules de l'os, les ostéoblastes.

Différents matériaux de substitution du tissu osseux ont déjà été proposes.

Conformément au document EP—A—0030583, on a proposé un matériau de substitution à structure lacunaire, composé de collagène et d'apatite, éventuellement renforcé par une structure artificielle du type nid d'abeille. Un tel matériau est propose pour remplir une cavité osseuse, et empêcher le saignement de l'os, tout en permettant un cicatrisation de ce dernier.

Des expériences intéressantes (Hayashi et al, Arch. Orhtop. traumat. et Surg. 99,265-269-1982) ont démontré qu'un tel matériau présentait une ostéogénèse relativement faible, de telle sorte qu'il doit être considéré essentiellement comme un matériau de remplissage, à des fins de prothèse ou chirurgie osseuse.

Conformément au document FR—A—2350 826, on a proposé un matériau de substitution composé de phosphate de calcium fritté et d'un polymère biodégradable, d'origine synthétique, ou d'origine naturelle, tel que du collagène tanné. Ces deux types de polymères d'avèrent n'avoir aucune influence favorable sur le développement des ostéoblastes. De plus, la dégradation de ces polymères dans l'organisme peut y introduire des résidus plus ou moins toxiques.

En dehors des matériaux de substitution du tissu osseux, d'autres matériaux ayant une composition hors matières minérales relativement proche, à savoir en collagène et glycosaminoglycanes, ont également été proposés.

Ainsi selon le document FR—A—2318 189, on a proposé un matériau associant un collagène et un glycosaminoglycane, par réticulation déhydrothermique, utilisable pour constituer des prothèses en contact avec le sang, telles que les veines et artères. Par des essais appropriés, la Demanderesse a démontré qu'un tel matériau ne peut constituer un produit de substitution du tissu osseux, en particulier parce qu'il ne conduit pas à une ostéogenèse en volume.

La présente invention a pour objet un véritable matériau de substitution du tissu osseux, c'est-à-dire un matériau bio-compatible favorisant l'ostéogenèse, au sens d'un matériau temporaire laissant progressivement la place à un tissu ostéoïde susceptible de prendre avec le temps les caractères mécaniques et histologiques du tissu osseux, notamment de la matrice osseuse.

Conformément à la présente invention, un matériau de substitution, se présentant avantageusement sous la forme d'éponge, comprend une association d'un glycosaminoglycane, par exemple l'acide chondroïtine-4-sulfate, lié au collagène par réticulation déhydrothermique, ainsi que de l'hydroxyapatite.

Préférentiellement, le collagène approprié à l'invention est du collagène déréticulé.

Un matériau selon l'invention peut être obtenu à partir d'un gel à 1% de collagène, comportant 20 g/l d'hydroxypatite, et 1,6 g/l de glycosaminoglycane.

La présente invention est maintenant décrite par référence aux exemples ci-après, qui l'illustrent sans toutefois la limiter.

### Exemple 1

Préparation d'une éponge collagène déréticulé-acide chondroïtine-4-sulfate-hydroxyapatite

a) Préparation de collagène déréticulé

Des peaux de veaux provenant d'animaux fraîchement abattus sont tout d'abord lavées à l'eau du robinet par brassage pendant une heure dans un foulon.

Le système pileux et le tissu sous-cutané sont séparés du derme à l'aide d'une refendeuse à bande rotative.

Le derme récupéré est haché et broyé. Le broyat est lavé par trois bains successifs de tampon phosphate (pH 7,8). Entre chaque bain, le broyat est séparé de la solution par centrifugation en continu à 4 000 t/minute. Le résidu est rincé par deux bains successifs d'eau permutée et le liquide est séparé du broyat de la même façon que dans les opérations précédentes. Ces premiers traitements servent à éliminer les substances non-collagéniques. Le tissu est alors placé dans une cuve contenant une solution de soude à pH 14. Après agitation pendant une demi-heure environ, l'ensemble est laissé au repos pendant huit jours. Le milieu est ensuite acidifié par l'acide chlorhydrique jusqu'à l'obtention d'un pH de 2. On ajoute au mélange obtenu du chlorure de sodium jusqu'à obtention d'une concentration de 10%. Le collagène précipité est dialysé contre de l'eau permutée stérile.

b) Préparation de l'acide chondroïtine-4-sulfate

Cet acide est extrait des cloisons nasales de veau. Celles-ci sont tout d'abord lavées soigneusement dans une solution de chlorure de sodium à 9 pour 1 000. Elles sont ensuite hachées et broyées. Le broyat est alors placé à raison de 1 kg par litre dans une solution de potasse à 0,5 N. Après agitation, l'ensemble est laissé au repos à température ambiante pendant 24 heures. Au bout de ce laps de temps, le surnageant est séparé des matières insolubles par centrifugation à 30 000 g pendant 50 minutes. De l'acide acétique pur est ajouté au surnageant pour neutraliser la soude. La solution est ensuite concentrée cinq fois par évaporation sous vide. Le concentrat est versé dans trois fois son volume d'éthanol. Le précipité, récupéré par décantation, est dissous dans l'eau permutée. L'acide chondroïtine-4-sulfate est obtenu par lyophilisation de cette solution.

c) Préparation de l'hydroxyapatite

Deux volumes V de solution 0,5 M de $CaCl_2$ et 0,5 M de $Na_2HPO_4$ sont versés sous agitation à des vitesses égales dans un bécher.

Le précipité formé est décanté et lavé quatre fois avec des volumes 2V d'eau. La suspension est diluée dans un volume 2V d'eau distillée et il lui est ajouté dans une proportion de 100 ml pour 4 l une solution fraîchement préparée de NaOH à 40%. Le mélange est maintenu à l'ébullition pendant une heure. Après décantation, le liquide surnageant est éliminé. Le précipité récupéré est lavé quatre fois par l'eau. Du tampon phosphate de sodium à 0,01 M à pH 6,8 est ajouté au précipité et la suspension est portée à ébullition. Après décantation, le surnageant est éliminé et du tampon frais est ajouté. La suspension est alors maintenue à ébullition pendant cinq minutes. Après décantation, le précipité est maintenu à ébullition dans le même tampon pendant 15 minutes et ensuite deux fois avec un tampon phosphate 0,001 M. Après élimination de l'eau l'hydroxyapatite, est obtenue sous forme de poudre.

d) Préparation du mélange et obtention de l'éponge

20 g d'hydroxyapatite et 1,6 g d'acide chondroï-tine-4-sulfate sont placés dans un litre de gel d'atelocollagène à 1% dans l'eau permutée à un pH de 6,5. Après homogénéisation, le mélange est versé dans un plateau, puis lyophilisé. Le lyophilisat est alors découpé en morceaux de dimensions 35×20×10 mm. Les éponges ainsi obtenues sont séchées sous vide de 0,1 mm de mercure pendant 48 heures à 80°C.

Exemple 2
Préparation d'une éponge collagène natif-acide chondroïtine-4-sulfate-hydroxyapatite
a) Préparation d'une suspension homogène de collagène

Des peaux de veaux provenant d'animaux fraîchement abattus sont lavées à l'eau du robinet par brassage pendant une heure dans un foulon. Le système pileux et le tissu sous-cutané sont séparés du derme à l'aide d'une refendeuse à bande rotative.

Le derme récupéré est hâché et broyé. Le broyat est lavé par trois bains successifs de tampon phosphate pH 7,8. Entre chaque bain, le broyat est séparé de la solution par centrifugation en continu à 4 000 t/minute. Le résidu est rincé par deux bains successifs d'eau permutée stérile et le liquide est séparé du broyat de la même façon que dans les opérations précédentes. Le tissu est alors placé dans une solution d'acide acétique. La concentration en collagène de cette solution doit être de 10% et celle de l'acide de 40% par rapport à la protéine.

La pâte obtenue est ensuite diluée par l'eau permutée stérile de façon à obtenir une concentration de 3% en collagène. Cette nouvelle préparation est homogénéisée dans un broyeur type Ultra-Turraux.

b) La préparation de l'acide chondroïtine-4-sulfate se fait dans les conditions indiquées dans l'exemple 1.

c) La préparation de l'hydroxyapatite se fait dans les conditions indiquées dans l'exemple 1.

d) Préparation du mélange et obtention de l'éponge

Dans le gel de collagène à 3 g sont ajoutés sous forme solide l'hydroxyapatite et le glycosaminoglycanne, la concentration finale pour la première substance étant égale à celle du collagène et celle de la deuxième substance étant six fois inférieure. Après une agitation d'une durée minimum heure, le mélange est centrifugé. Le culot est ensuite placé dans un plateau ou dans un récipient de forme adéquate et lyophilisé. La dureté du matériau obtenu variera dans le même sens que la vitesse et la durée de la centrifugation.

Expérimentation des éponges collagène-hydroxyapatite-glycosaminoglycannes

Une expérimentation animale a été réalisée en utilisant un modèle de pseudoarthrose expérimental. Ce travail a consisté à enlever chez des chiens 20 à 25 mm de portions de fémur et à combler l'espace ainsi libéré à l'aide de plusieurs éprouvettes du biomatériau à tester. La rigidité de l'ensemble a été assurée par une plaque métallique vissée dans les deux parties de l'os rendues non solidaires. Certains chiens ont subi une trépanation du grand trochanter à l'aide d'un foret et d'une fraise de façon à pouvoir implanter un morceau d'obturateur médullaire constitué de collagène acido soluble d'hydroxyapatite et de glycosaminoglycanne. D'autres chiens ont servi de témoins. Dans ce but, ils ont reçu en implantation dans la perte de substance diaphysaire de l'os spongieux prélevé sur la crête iliaque homolatérale. Le lieu de prélèvement a été comblé par les éprouvettes atélocollagène hydroxyapatite-glycosaminoglycannes. Les animaux ont été sacrifiés à des temps variant entre un et six mois après les implantations des éprouvettes.

Les résultats de cette expérimentation ont montré qu'il n'y avait pas de réaction inflammatoire aigüe vis-à-vis du biomatériau et qu'aucune réaction immunitaire ne semblait se manifester vis-à-vis du collagène. On a pu constater un comblement de la perte de substance et, dans certains cas, un agrafage osseux avec un aspect radiologique homogène dense avec, à l'histologie, un véritable front d'ossification.

De plus, les éprouvettes semblent empêcher l'apparition d'une fibrose cicatricielle qui s'oppose par substitution progressive à la consolidation. Enfin, elles évitent la constitution d'une pseudoarthrose, si le montage os-biomatériau reste solide.

Le biomatériau décrit ci-avant présente donc un grand intérêt dans la chirurgie de l'appareil locomoteur et reconstructif.

**Revendications**

1. Matériau de substitution de la matrice osseuse, favorisant l'ostéogénèse, caractérisé en ce qu'il comprend une association d'un glycosaminoglycane lié au collagène par réticulation déhydrothermique, ainsi que de l'hydroxyapatite.

2. Matériau selon la revendication 1, caractérisé en ce que la collagène est du collagène déréticulé.

3. Matériau selon la revendication 1, caractérisé en ce que le glycosaminoglycane est l'acide chondroïtine-4-sulfate.

4. Procédé d'obtention d'un matériau selon l'une quelconque des revendications 1 à 3, caractérisé en ce qu'on part d'un gel à 1% de collagène, comportant 20 g/l d'hydroxyapatite, et 1,6 g/l de glycosaminoglycane.

**Patentansprüche**

1. Ersatzmaterial für die Knochenmatrix, das die Osteogenese fördert, dadurch gekennzeichnet, daß es eine Assoziation eines an Kollagen durch dehydrothermische Vernetzung gebundenes Glykosaminoglykan sowie Hydroxyapatit enthält.

2. Material nach Anspruch 1, dadurch gekennzeichnet, daß das Kollagen entnetztes Kollagen ist.

3. Material nach Anspruch 1, dadurch gekennzeichnet, daß das Glykosaminoglykan Chondroitin-4-sulfat ist.

4. Verfahren zum Erhalten eines Materials nach einem der Ansprüche 1 bis 3, dadurch gekennzeichnet, daß von einem Gel mit 1% Kollagen ausgegangen wird, das 20 g/l Hydroxyapatit und 1,6 g/l Glykosaminoglykan enthält.

**Claims**

1. Replacement material for the bone matrix, promoting osteogenesis, characterized in that it comprises a combination of a glycosaminoglycan linked to collagen by dehydrothermic crosslinking, as well as hydroxyapatite.

2. Material according to Claim 1, characterized in that the collagen is decrosslinked collagen.

3. Material according to Claim 1, characterized in that the glycosaminoglycan is chondroitin-4-sulphate acid [sic].

4. Process for obtaining a material according to any one of Claims 1 to 3, characterized in that the starting material is a 1% collagen gel containing 20 g/l of hydroxyapatite and 1.6 g/l of glycosaminoglycan.